# EUROPEAN PATENT SPECIFICATION

(11) **EP 0 926 143 B1**
(45) Date of publication and mention of the grant of the patent: **07.09.2005**
(21) Application number: 98122762.2
(22) Date of filing: 01.12.1998
(51) Int. Cl.: C07D 285/12

(54) **Synthesis of 2-(methylsulfonyl)-5-(trifluoromethyl)-1,3,4-thiadiazole in the presence of a molybdenum or tungsten catalyst**
Synthese von 2-(Methylsulfonyl)-5-(Trifluormethyl)-1,3,4-Thiadiazol in Abwesenheit eines Molybdän- oder Wolfram-Katalysators
Synthèse de 2-(methylsulfonyl)-5-(trifluormethyl)-1,3,4-thiadiazole en présence de catalyseur au molybdène ou tungstène

(30) Priority: 12.12.1997 US 989568
(43) Date of publication of application: 30.06.1999
(73) Proprietor: BAYER CORPORATION, Pittsburgh, PA 15205-9741 (US); Bayer Aktiengesellschaft, 51368 Leverkusen (DE)
(72) Inventor: Desai, Vijay C., Shawnee, KS 66216 (US); Erdman, David T., 51061 Köln (DE); Applegate, Jacqueline M. Dr., 51377 Leverkusen (DE); Jelich, Klaus Dr., Overland Park, KS 66223 (US); Noack, Achim, 42799 Leichlingen (DE)
(74) Representative: Schwenk, Norbert

(56) References cited:
- EP-A- 0 165 537
- EP-A- 0 298 338
- DE-A- 3 722 320
- US-A- 5 856 499

## Description

### TECHNICAL FIELD OF THE INVENTION

The field of the present invention is the synthesis of thiadiazole sulfones. More particularly, the present invention pertains to the synthesis of 2-(methylsulfonyl)-5-(trifluoromethyl)-1,3,4-thiadiazole using catalytic oxidation.

### BACKGROUND OF THE INVENTION

Sulfones have the general structure R-SO₂-R'. Sulfones can be produced from a variety of precursors. By way of example, sulfones can be prepared by (a) oxidizing sulphides, (b) rearranging sulphinate esters, (c) adding sulfonyl halides to alkenes and acetylenes, (d) adding sulphinic acids to polarized bonds, and (e) adding (cyloaddition) of SO₂ to polyenes (See, e.g., Durst, T., in Comprehensive Organic Chemistry: Chapter 11.6, Barton and Ollis, Eds., Pergammon Press, Oxford, 1979).

A particular class of sulfones, 2-(alkylsulfony)-5-(trifluoromethyl)-1,3,4-thiadiazoles are intermediates used in the production of herbicides. 2-(methylsulfonyl)-5-(trifluoromethyl)-1,3,4-thiadiazole has been reported to possess antifungal activity (See, U.S. Patent 3,562,284). According to U.S. Patent 3,562,284, 2-(substituted sulfonyl)-5-(trifluoromethyl)-1,3,4-thiadiazoles can be made by oxidizing a corresponding 2-(substituted thio)-5-(trifluoromethyl)-1,3,4-thiadiazole in the presence of an oxidizing agent such as potassium permanganate, hydrogen peroxide or peroxytrifluoroacetic acid. Oxidation takes place in an acidic, aqueous medium that includes acetic acid and methylene chloride as a solvent. Methylene chloride is an undesirable solvent from the standpoint of industrial hygiene and handling. It is difficult to handle due to the low boiling point (high vapor pressure) and it also contaminates aqueous waste streams. The sulfone product is isolated using crystallization. The reported yield of the sulfone, based on the starting sulfide, was about 65%.

The use of acetic acid in the presence of water in the process necessarily introduces excess water into the reaction and requires purification of the sulfone using expensive crystallization procedures with resultant low yields. There continues to be a need in the art for a practical, inexpensive process for preparing thiadiazole sulfones in high yield.

### BRIEF SUMMARY OF INVENTION

The present invention provides a process for making 2-(methylsulfonyl)-5-(trifluoromethyl)-1,3,4-thiadiazole. The process includes the step of oxidizing 2-(methylthio)-5-(trifluoromethyl)-1,3,4-thiadiazole in a reaction mixture containing an activated molybdenum or tungsten catalyst to form a reaction product. According to the invention 2-(methylthio)-5-(trifluoromethyl)-1,3,4-thiadiazole is reacted with hydrogen peroxide in the presence of the activated catalyst. The 2-(methylthio)-5-(trifluoromethyl)-1,3,4-thiadiazole is dissolved in an aprotic, aromatic solvent such as toluene.

The activated catalyst is molybdic acid or tungstic acid. Such an acid can be directly added to the reaction mixture or can be formed in the reaction mixture from tungstate or molybdate salts. The acid is preferably formed by acidifying an alkali metal molybdate or tungstate salt. A preferred alkali metal is sodium and a preferred acid for acidification is sulfuric acid. Formation of the activated catalyst can take place in the mixture. In accordance with this embodiment, 2-(methylthio)-5-(trifluoromethyl)-1,3,4-thiadiazole is reacted with hydrogen peroxide in the presence of the alkali metal salt and sulfuric acid.

The hydrogen peroxide used in the reaction mixture is preferably an aqueous solution containing from about 30 weight percent to about 50 weight percent hydrogen peroxide. The activated catalyst is present in an amount of from about 0.5 grams to about 10 grams of catalyst per mole of 2-(methylthio)-5-(trifluoromethyl)-1,3,4-thiadiazole. Oxidation typically occurs at a temperature of from 50°C to 100°C and, preferably at a temperature of from 70°C to 90°C.

The process of the present invention can include additional steps. Unspent catalyst can be recovered and the recovered catalyst optionally recycled into the reaction mixture. Further, water can be removed from the reaction product. The removal of water is preferably accomplished azeotropically. Still further, a process of this invention can include the step of isolating the formed 2-(methylsulfonyl)-5-(trifluoromethyl)-1,3,4-thiadiazole.

### DETAILED DESCRIPTION OF THE INVENTION

### I. The Invention

The present invention provides the process for producing thiadiazole sulfone. The present processes are used to make 2-(methylsulfonyl)-5-(trifluoromethyl)-1,3,4-thiadiazole (TDA sulfone) from 2-(methylthio)-5-(trifluoromethyl)-1,3,4-thiadiazole (TDA). TDA sulfone is made using catalytic oxidation of TDA in the presence of a suitable oxidizing agent. The catalyst used for the oxidation reaction is a molybdenum or tungsten catalyst.

### II. Process for Producing TDA Sulfone Using Tungsten or Molybdenum

In accordance with this aspect, the present process includes the step of oxidizing 2-(methylthio)-5-(trifluoromethyl)-1,3,4-thiadiazole (TDA) in a reaction mixture containing an activated molybdenum or tungsten catalyst to form a reaction product that contains the TDA sulfone product. Oxidation of TDA takes place in the presence of a hydrogen peroxide. The oxidizing agent is hydrogen peroxide (HO₂). In accordance with the invention, TDA is reacted with hydrogen peroxide in the presence of a catalyst. The hydrogen peroxide used in the reaction mixture is preferably an aqueous solution containing from about 30 weight percent to about 50 weight percent hydrogen peroxide. The molar ratio of H₂O₂ to TDA is from 2:1 to 4:1 and, preferably 2.2:1. Oxidation conditions are well known in the art. Typically, oxidation is carried out at a temperature of from 60°C to 100°C.

TDA used in a present process can be obtained from any source. According to the invention, the TDA is made by a process that provides TDA in an aprotic, aromatic solvent such as toluene. Especially preferred means for making TDA can be found in the U.S. Patent Applications entitled "A Process for Making 2-(Methylthio)-5-(Trifluoromethyl)-1,3,4-Thiadiazole Using Methyldithiocarbazinate and a Molar Excess of Trifluoroacetic Acid", "A Process of Making 2-(Methylthio)-5-(Trifluoro-methyl)-1,3,4-Thiadiazole Using Methyldithiocarbazinate with Trifluoro-acetic Acid with Removal of Bis-Byproduct", and "A Process for Preparing 2-(Methylthio)-5-(Trifluoromethyl)-1,3,4-Thiadiazole Using Methyldithiocarbazinate and a Molar Excess of Trifluoroacetic Acid With Recovery of Trifluoroacetic Acid", filed concurrently herewith and published as U.S. patents Nos. 5,905,157, 6,005,114, and 5,898,074. The disclosures of all three applications are incorporated herein by reference.

The oxidation of TDA occurs in the presence of a solvent. The solvent is an aprotic, aromatic solvent. Such solvents are well known in the art. Exemplary and preferred such solvents are toluene, xylene, cumene and mesitylene. Toluene is especially preferred. The amount of solvent used can vary over a wide range as readily determined by a skilled artisan. The precise amount of solvent will depend on the particular solvent used. Where toluene is the solvent, it is present in an amount from 0.5 moles to 3.5 moles of toluene per mole of TDA. Preferably, toluene is present in an amount of from 1.0 moles to 2.0 moles per mole of TDA and, more preferably, in an amount of from 1.0 to 1.5 moles of toluene per mole of TDA.

The term "activated catalyst" as used herein means molybdic acid or tungstic acid. Such acids include acid salts such as ammonium tungstate or ammonium molybdate. The activated catalyst is present in an amount of from 0.005 to 0.035 moles of catalyst per mole of TDA. Preferably, the molar ratio of catalyst to TDA is 0.015:1. Such an acid can be directly added to the reaction mixture or can be formed in the reaction mixture from tungstate or molybdate salts. The acid is preferably formed by acidifying an alkali metal molybdate or tungstate salt. Exemplary and preferred such alkali metal salts are well known in the art and are commercially available. Especially preferred alkali metal salts are sodium, potassium and lithium salts of tungstic or molybdic acid. Sodium salts such as Na₂ (MoO₄) and Na₂(WO₄) are most preferred.

The activated catalyst is formed by acidifying the alkali metal salt with an acid. Exemplary and preferred acids are inorganic acids having a pKa between about 1 and 4. Especially preferred acids are hydrochloric acid, sulfuric acid and nitric acid. Sulfuric acid (H₂SO₄) is most preferred. Formation of the activated catalyst can take place in the oxidation reaction mixture itself. In accordance with this embodiment, TDA is reacted with hydrogen peroxide in the presence of the alkali metal salt and acid.

Even where the acid catalyst (e.g., molybdic acid) is added directly to the reaction mixture, the reaction mixture can further contain additional acid. That acid can be an inorganic acid as set forth above or trifluoro-acetic acid. The acid is present in an amount of from about 0.01 to about 0.03 moles per mole of TDA.

A process for the present invention as set forth in Section II above, can include additional steps. The catalyst can be recovered and the recovered catalyst optionally recycled into the reaction mixture. Further, water can be removed from the reaction product. Still further, a process of this invention can include the step of isolating the formed sulfone.

Water removal is preferably accomplished azeotropically. The azeotropic removal of water is readily accomplished in the presence of the solvent, particularly where the solvent is toluene. Because the azeotrope has a lower boiling point than water, heating the reaction product to the boiling point of the solvent effectively removes the water. Because the oxidation reaction occurs in the range of 60°C to 85°C, water is removed during the reaction; no additional step is required.

The following examples illustrate preferred embodiments of the present invention and are not limiting of the specification and claims in any way.

### EXAMPLES

### Example 1: Synthesis of TDA Sulfone Using A Molydenum Catalyst

5 grams of sodium molybdate monohydrate and 2 grams of concentrated H₂SO₄ were added to a solution of 2-(methylthio)-5-(trifluoromethyl)-1,3,4-thiadiazole (TDA) (202.6 g of 98.8% purity, 1.0 mole) in toluene (116.2 g) with stirring. The mixture was heated to 80°C with stirring. A 35 weight percent H₂O₂ solution in water (260 g; 2.67 mole) was added uniformly over 4 hours. During the addition of H₂O_{2,} the reaction temperature was maintained at 80±2°C.

The reaction mixture was cooked at 80°C until mono-oxidized species of TDA were less than 1 % (as analyzed by gas chromatography) (approx. 2-3 hours). Water was removed azeotropically with toluene. The reaction mixture was diluted with about 225 grams of toluene and the solid filtered out at 60°C using steam jacketed suction filtration. The TDA sulfone was isolated from the filtrate (purity 98%, net yield 95.5%).

### A. Recycle of Catalyst

The filtered out solid from above and 2.0 grams concentrated H₂SO₄ was added to a solution of TDA (202 g of 98.8% purity; 1.0 mole) in toluene (116.2 g) with stirring. 35% aqueous H₂O₂ (260 g; 2.67 mole) was added uniformly over 4 hours. During the addition of H₂O₂, the temperature was maintained at 80±2°C. The reaction mixture was cooked at 80°C until the mono-oxidized species of TDA was less than 1 % (as analyzed by gas chromatography) (approx. 2-3 hours).

Reaction water was removed azeotropically with toluene. The reaction mixture was then diluted with about 225 grams of toluene and the solids filtered out at 60°C using suction filtration. The cake was saved for recycle. The TDA-sulfone was isolated from the filtrate (purity 98.1%, net yield 98%). The sodium molybdate catalyst can be used in the molar ratio of 3-5 grams of catalyst per mole TDA.

### B. Use of Sulfuric Acid

In another series of studies, 5 grams of molybdic acid, in the absence or presence of H₂SO₄, was used as a catalyst. Catalyst was recycled numerous times. Subsequent recycles were done using molybdic acid and H₂SO₄ from the preceding run. The reaction conditions and the stoichiometry used in each series were as follows:

| | Series A (no H₂SO₄) | Series B (with H₂SO₄) |
|---|---|---|
| Molybdic acid | 5 g | 5 g |
| Conc. H₂SO₄ | --- | 3.75 g |
| Conc of TDA in toluene | 60% | 60% |
| TDA | 1 mol | 1 mol |
| Reaction Temperature | 80°C | 80°C |
| H₂O₂ Add Time | 3 hrs | 3 hrs |
| Cook Time (<1% sulfoxide) | 2-3 hrs | 2-3 hrs |
| Excess H₂O₂ | 30-60% | 30-60% |

Water was azeotropically removed from the reaction product. The organic layer was diluted down to 40% TDA-SO₂ in toluene from 60% TDA-SO₂ in toluene. The analytical results are summarized below:

| | %Sulfone | % Net Yield | % Sulfoxide |
|---|---|---|---|
| Series A (Molybdic acid alone) | 98.6 | 94.9 | 0.6 |
| Series B (Molybdic acid + H₂SO₄) | 96.8 | 91.7 | 0.5 |

### C. Use of Ammonium Molybdate

Two TDA-sulfone batches (original + 1 recycle) were made using ammonium dimolybdate as the catalyst. Ammonium dimolybdate is the major constituent of molybdic acid. The physical and chemical characteristics of the oxidation reactions were identical with that of reactions using molybdic acid as the catalyst. The net yields using ammonium molybdate were both in the 98-99% range.

### Example 2: Synthesis of TDA Sulfone Using Tungsten Catalyst

TDA in toluene and sodium tungstate (Na₂WO₄) were charged in a reaction vessel. The catalyst was either fresh or recycled from a previous reaction batch. The mixture was heated to the reaction start temperature. 35 weight percent H₂O₂ was added over time. The reaction mixture was then heated to the reaction temperature for 2-3 hours. The mixture was N₂ purged during the reaction. Reaction conditions were as follows:

| | |
|---|---|
| TDA/Toluene, wt. % TDA | 57-60 |
| Reaction start temperature, °C | 70-74 |
| H₂O₂/TDA Mole Ratio | 2.30-2.40 |
| H₂O₂ add time, hrs | 3.0-3.5 |
| Reaction temperature, °C | 80-90 |
| Reaction time, hrs | 2.0-3.0 |

The reaction product mixture was then heated to reflux and some water was removed by azeotropic distillation. The mixture was cooled to 70°C and the phases are separated.

The aqueous catalyst phase was retained for recycle to the next batch. The TDA sulfone/toluene phase was retained for subsequent use.

The average net yield for the sodium tungstate process was about 97.5%.

Although the invention has been described in detail in the foregoing for the purpose of illustration, it is to be understood that such detail is solely for that purpose and that variations can be made therein by those skilled in the art.

## Claims

1. A process for making a 2-(methylsulfonyl)-5-(trifluoro-methyl)-1,3,4-thiadiazole comprising oxidizing 2-(methylthio)-5-(trifluoromethyl)-1,3,4-thiadiazole with hydrogen peroxide in an aprotic, aromatic solvent containing an activated molybdenum or tungsten catalyst to form a reaction product.

2. The process of Claim I wherein the solvent is toluene.

3. The process of Claim 1 wherein oxidation occurs at a temperature of from 50°C to 100°C.

4. The process of Claim 1 wherein the hydrogen peroxide is an aqueous solution containing from 30 weight percent to 50 weight percent hydrogen peroxide.

5. The process of Claim 1 wherein the activated catalyst is present in amount of from 0,5 grams to 10 grams of catalyst per mole of 2-(methylthio)-5-(trifluoromethyl)-1,3,4-thiadiazole.

6. The process of Claim 1 further comprising azeotropically removing water from the reaction product.

7. The process of Claim 1 further comprising revovering the formed 2-(methylsulfonyl)-5-(trifluoromethyl)-1,3,4-thiadiazole.

## Patentansprüche

1. Verfahren zur Herstellung von 2-(Methylsulfonyl)-5-(trifluormethyl)-1,3,4-thiadiazol, darin bestehend, dass 2-(Methylthio)-5-(trifluormethyl)-1,3,4-thiadiazol mit Wasserstoffperoxid in einem aprotischen aromatischen Lösemittel, das einen aktivierten Molybdän-oder Wolfram-Katalysator enthält, oxidiert wird, um ein Reaktionsprodukt zu erhalten.

2. Verfahren nach Anspruch 1, bei dem das Lösemittel Toluol ist.

3. Verfahren nach Anspruch 1, bei dem die Oxidation bei einer Temperatur von 50°C bis 100°C stattfindet.

4. Verfahren nach Anspruch 1, bei dem das Wasserstoffperoxid eine wässrige Lösung mit 30 Gew.-% bis 50 Gew.-% Wasserstoffperoxid ist.

5. Verfahren nach Anspruch 1, bei dem der aktivierte Katalysator in einer Menge von 0,5 Gramm bis 10 Gramm Katalysator pro Mol 2-(Methylthio)-5-(trifluormethyl)-1,3,4-thiadiazol vorliegt.

6. Verfahren nach Anspruch 1, das zusätzlich die azeotrope Entfernung von Wasser aus dem Reaktionsprodukt umfasst.

7. Verfahren nach Anspruch 1, das zusätzlich die Gewinnung des gebildeten 2-(Methylsulfonyl)-5-(trifluormethyl)-1,3,4-thiadiazols umfasst.

## Revendications

1. Procédé de préparation d'un 2-(méthylsulfonyl)-5-(trifluorométhyl)-1,3,4-thiadiazole comprenant l'oxydation du 2-(méthylthio)-5-(trifluorométhyl)-1,3,4-thiadiazole par le peroxyde d'hydrogène dans un solvant aromatique aprotique, renfermant un catalyseur activé au molybdène ou au tungstène, en vue de former un produit réactionnel.

2. Procédé selon la revendication 1, où le solvant est le toluène.

3. Procédé selon la revendication 1, où l'oxydation se produit à une température de 50°C à 100°C.

4. Procédé selon la revendication 1, où le peroxyde d'hydrogène est une solution aqueuse renfermant de 30 % en masse à 50 % en masse de peroxyde d'hydrogène.

5. Procédé selon la revendication 1, où le catalyseur activé est présent en une quantité de 0,5 g à 10 g de catalyseur par mole de 2-(méthylthio)-5-(trifluorométhyl)-1,3,4-thiadiazole.

6. Procédé selon la revendication 1, comprenant de plus l'élimination azéotropique de l'eau à partir du produit réactionnel.

7. Procédé selon la revendication 1, comprenant de plus la récupération du 2-(méthylsulfonyl)-5-(trifluorométhyl)-1,3,4-thiadiazole formé.
